# EUROPÄISCHE PATENTSCHRIFT

(11) **EP 3 944 813 B1**
(45) Veröffentlichungstag und Bekanntmachung des Hinweises auf die Patenterteilung: **05.08.2026**
(21) Anmeldenummer: 21187819.4
(22) Anmeldetag: 26.07.2021
(51) Int. Cl.: A61B 5/11, A61B 5/0536

(54) **VORRICHTUNG UND VERFAHREN ZUR ERKENNUNG UND VORHERSAGE VON KÖRPERBEWEGUNGEN**
METHOD AND DEVICE FOR IDENTIFYING AND PREDICTING MOVEMENTS OF A BODY
DISPOSITIF ET PROCÉDÉ DE DÉTECTION ET DE PRÉVISION DES MOUVEMENTS CORPORELS

(30) Priorität: 28.07.2020 DE 102020119907
(43) Veröffentlichungstag der Anmeldung: 02.02.2022
(73) Patentinhaber: Enari GmbH, 85748 Garching b. München (DE)
(72) Erfinder: Rettlinger, Sebastian, 85540 Haar (DE)
(74) Vertreter: Lermer, Christoph

(56) Entgegenhaltungen:
- WO-A1-2020/102693
- US-A1- 2016 038 083
- US-A1- 2017 061 817
- US-A1- 2017 123 487
- US-A1- 2017 238 812
- WU YU ET AL: "A Human-Machine Interface Using Electrical Impedance Tomography for Hand Prosthesis Control", IEEE TRANSACTIONS ON BIOMEDICAL CIRCUITS AND SYSTEMS, IEEE, US, vol. 12, no. 6, 1 December 2018 (2018-12-01), pages 1322 - 1333, XP011695336, ISSN: 1932-4545, [retrieved on 20190101], DOI: 10.1109/TBCAS.2018.2878395

## Beschreibung

### TECHNISCHES GEBIET

Die Anmeldung betrifft ein System zur Erkennung und Vorhersage von Bewegungen eines Benutzers, umfassend einen Detektionsbereich mit mindestens einem Sensor, einen Modellierungsbereich und einen Aktuator, wobei der Sensor geeignet ist, über den Körperzustand des Benutzers Messdaten zu erheben und an den Modellierungsbereich weiterzuleiten. Außerdem betrifft die Anmeldung Verfahren zur Erkennung und Vorhersage von Bewegungen eines Benutzers.

### STAND DER TECHNIK

Es gibt eine Vielzahl von komplexen Bewegungsabläufen des Köpers eines Benutzers, die ein hohes Maß an Körperbeherrschung erfordern und zu deren Erlangung ein entsprechendes intensives Training erforderlich ist. Hierfür gibt eine Vielzahl von technischen Vorrichtungen und Verfahren zur Überwachung und Verbesserung des Trainingseffekts, mittels derer eine Erfassung von Messdaten, eine Verarbeitung von Daten und eine Rückmeldung über den aktuellen Bewegungszustand des Körpers möglich ist.

Verfahren zur Erkennung und Vorhersage von Bewegungen sind aus den folgenden Druckschriften bekannt: WO-2020/102693-A1, US-2017/061817-A1, US-2017/123487-A1, US-2017/238812-A1, US-2016/038083-A1, und WU YU ET AL: "A Human-Machine Interface Using Electrical Impedance Tomography for Hand Prosthesis Control", IEEE TRANSACTIONS ON BIOMEDICAL CIRCUITS AND SYSTEMS, IEEE, US, Bd. 12, Nr. 6, Dezember 2018, ISSN: 1932-4545, DOI: 10.1109/TBCAS.2018.2878395.

Allerdings sind die Möglichkeiten der Analyse und sich daraus ergebender Trainingsanweisungen auf Basis der Visualisierung aktueller Bewegungszustände beschränkt. Insbesondere kann zwar eine Visualisierung des Bewegungszustands ohne größere Zeitverzögerungen erfolgen. Das Ziehen von Rückschlüssen auf das Trainingsprogramm erfordert jedoch eine gesonderte Auswertung.

### AUFGABE DER ERFINDUNG

Ausgehend davon besteht die Aufgabe der vorliegenden Erfindung darin, ein System und Verfahren bereitzustellen, welche die Messdaten über den Körperzustand eines Benutzers erheben und derart verarbeiten, dass eine latenzfreie und zuverlässige Bereitstellung von Trainingsanweisungen bzw. Trainingsdaten ermöglicht wird.

### TECHNISCHE LÖSUNG

Diese Aufgabe wird durch ein System nach Anspruch 1 und ein Verfahren nach Anspruch 11 gelöst.

Das beschriebene System zur Erkennung und Vorhersage von Bewegungen eines Benutzers umfasst einen Detektionsbereich mit mindestens einem Sensor einen Modellierungsbereich und einen Aktuator, wobei der Sensor geeignet ist, über den Körperzustand des Benutzers Messdaten zu erheben und an den Modellierungsbereich weiterzuleiten, wobei die Erfassung des Körperzustands des Benutzers mittels EIT (elektrische Impedanz Tomographie) und/ oder EMG (Elektromyographie) und/ oder UWB (Ultra-wideband) Echtzeit-Lokalisierung erfolgt, und wobei der Modellierungsbereich geeignet ist, aufgrund der Messdaten durch Machine Learning ein Datenverarbeitungsmodel zur Berechnung eines aktuellen und/oder zukünftigen Körperzustands des Benutzers zu erstellen, und wobei der Aktuator geeignet ist, auf Basis des Datenverarbeitungsmodels auf den Körper des Benutzers und/oder auf eine vom Benutzer verwendete Vorrichtung einzuwirken..

Der Detektionsbereich dient als Benutzerebene, in der der Sensor alleine oder als Teil einer Sensorik kompakt an dem Benutzer selbst und/oder einem Interaktionsgegenstand, oder in deren Messumgebung angebracht und integriert ist. Im Detektionsbereich findet die Datenakquise, also die Erhebung von Messdaten, statt.

Bereits bei der Erhebung der Daten kann eine Reduzierung der Messdaten bzw. eine minimale Vorverarbeitung (z.B. Filtern, Komprimieren, Fusionieren) stattfinden. Z.B. können Messwerte, die außerhalb eines zulässigen Korridors relevanter Messdaten liegen, und/oder auf die von vornherein verzichtet werden kann, bereits im Messprozess unberücksichtigt bleiben. Ziel ist hierbei eine Größenreduktion der Messdaten. Hierbei werden Messdaten komprimiert, um deren Umfang zu reduzieren.

Die Daten werden im Anschluss an den Modellierungsbereich übertragen.

Im Modellierungsbereich werden die Messdaten dazu verwendet, durch Machine Learning ein Datenverarbeitungsmodel zu erstellen.

Machine Learning bzw. Maschinelles Lernen als Teilbereich der künstlichen Intelligenz ist die Generierung von Wissen aus Erfahrung eines maschinellen, künstlichen Systems. Dieses lernt auf Basis vorhandener Datenbestände und aus Beispielen und kann diese nach Beendigung der Lernphase verallgemeinern. Dazu bauen Algorithmen beim maschinellen Lernen ein statistisches Modell auf, das auf Trainingsdaten, im vorliegenden Fall den bereitgestellten Messdaten, beruht. Das heißt, es werden nicht einfach die Beispiele auswendig gelernt, sondern Muster und Gesetzmäßigkeiten in den Lerndaten erkannt. So kann das System auch unbekannte Daten beurteilen (Lerntransfer).

Das Machine Learning kann durch Analysen in Convolutional Neural Networks (CNN) erfolgen.

Ein Convolutional Neural Network (CNN oder ConvNet) ist ein künstliches neuronales Netz. Es handelt sich um ein von biologischen Prozessen inspiriertes Konzept zur maschinellen Verarbeitung von Bild- oder Audiodaten. Das Ergebnis des Machine Learning Prozesses ist ein Profiling & Anticipation (Profilierungs- und Vorhersage-)Modell, mit dessen Hilfe im Gebrauch des Systems Bewegungsdatensätze errechnet werden.

Im Grundsatz werden somit über den Sensor/ die Sensorik Bewegungen bzw. entsprechende Messdaten des Benutzers erfasst und durch den Modellierbereich profiliert, so dass auf Basis des Datenverarbeitungsmodells auch zukünftige Bewegungen vorausgesagt werden können.

Die Erfassung des Körperzustands des Benutzers kann mittels CS (compressed sensing) erfolgen.

Um große (Mess-) Datenmengen nicht zu stark anwachsen zu lassen, werden Daten häufig in komprimierter Form gespeichert, um den zur Archivierung der Daten benötigten Speicherplatz zu reduzieren. Als Standard für die Komprimierung von Bilddaten ist unter anderem die Norm ISO/IEC 10918-1, kurz als JPEG-Norm (Joint Photographic Experts Group) bezeichnet, gängig. Prinzipiell ist nach der JPEG-Norm eine verlustfreie Kompression (JPEG Lossless Mode) möglich.

Die Messdaten können bereits bei der Erfassung, das heißt unmittelbar bei der Akquirierung der Rohdaten, reduziert werden. Derartige Verfahren werden als CS-Verfahren ("Compressed Sensing" oder "Compressed Sampling") bezeichnet. CS-Verfahren legen einen Komprimierungsschritt mit dem Schritt der Datenaufnahme zusammen, indem - soweit möglich - nur relevante Daten des betrachteten Objektes gesammelt werden. Bei dem CS-Verfahren sorgt die während der Abtastung durchgeführte Komprimierung dafür, dass trotzt einer deutlichen Unterabtastung eine sehr hohe Bildqualität gesichert ist.

Eine Besonderheit von Daten, die mit CS-Verfahren aufgenommen wurden, liegt darin, dass die aufgenommenen Eingangsdaten einen geringeren Umfang haben können als weiterverarbeitete, entpackte Ausgangsdaten, welche an die Stelle von üblicherweise komprimierten Ausgangsdaten treten.

Der Aktuator kann jede Vorrichtung sein, die geeignet ist, auf Basis des Datenverarbeitungsmodels auf den Körper des Benutzers und/oder auf eine vom Benutzer verwendete Vorrichtung einzuwirken. Dies kann durch unmittelbare Einflussnahme z.B. durch haptische Signale oder mittelbar, z.B. durch optische oder akustische Signale, geschehen.

In einer bevorzugten Ausführung dient EIT der Erfassung der Muskelaktivität und/oder des Zustands des Körpergewebes, und die Detektion mittels EMG der Erfassung der Muskelaktivität und/oder des Zustands des Körpergewebes, und UWB Echtzeit-Lokalisierung der Erfassung von Gelenkstellungen und/oder der räumlichen Anordnung der Knochen des Benutzers.

EIT ist ein bildgebendes Verfahren, das auf Messungen infolge Impedanz- und Kapazitätsänderungen im menschlichen Körper basiert. Im Vergleich zu anderen tomographischen Verfahren, wie CT-Scans (Röntgenstrahlen), PET-Scans (Gammastrahlen), ist EIT nicht-invasiv. Die Sensoren ruhen auf der Haut, ohne z. B. leitfähiges Gel zu benötigen). EIT ist nicht invasiv und für Dauerbetrieb (z. B. niedrige Spannung, keine ionisierende Strahlung) geeignet. EIT wird im medizinischen Bereich z.B. zur Beurteilung der Herzfunktion, der pulmonalen Hypertonie und der regionalen Lungenfunktion eingesetzt.

Bei tomographischen Verfahren werden die innere Struktur und ggf. Zusammensetzung von Objekten durch die Verwendung von externen Signalen und deren Veränderung im Zeitablauf. EIT verwendet Oberflächenelektroden und hochfrequente Wechselstromsignale zur Messung der internen elektrischen Impedanz. Bei Verwendung mehrerer Elektroden kann eine interne Impedanzverteilung ermittelt werden. Da unterschiedliche Materialien innerhalb eines Objekts auf Signale unterschiedlich reagieren, kann die Querschnittsinnenstruktur des Objekts basierend auf den Variationen innerhalb der Impedanzverteilung rekonstruiert werden.

Mehrere Oberflächenelektroden werden um eine bestimmte Körperregion auf der Haut positioniert und zwischen jeweils zwei Elektroden fließen höherfrequente Wechselströme mit niedriger Amplitude während man simultan anhand der anderen Elektroden das elektrische Potential registriert. Mittels wiederholter Messungen bei beliebiger Variation des Stimulationselektrodenpaars wird ein Schnittbild (Tomogramm) erzeugt, aus dem Rückschlüsse über die Gewebezusammensetzung innerhalb der untersuchten Körperregion gezogen werden können.

Auf diese Weise werden mehrere Einzelbilder aus unterschiedlichen Schichten des zu betrachtenden Objekts erstellt und zu einem detaillierten dreidimensionalen Gesamtbild zusammengeführt.

EMG ist eine elektrophysiologische Methode, bei der die elektrische Muskelaktivität anhand von Aktionsströmen der Muskeln gemessen und ggf. (graphisch) dargestellt wird.

Mit Hilfe von konzentrischen Nadelelektroden oder auf der Haut aufliegenden Elektroden lassen sich die Potentialschwankungen einzelner motorischer Einheiten und der Haut mit Oberflächenelektroden erfassen.

UWB ist eine Ultra-Breitband-Technologie (engl.: Ultra-wideband) und ist eine Form der Nahbereichsfunkkommunikation bei Nutzung extrem großer Frequenzbereiche mit
einer Bandbreite von mindestens 500 MHz oder von mindestens 20 % des arithmetischen Mittelwertes von unterer und oberer Grenzfrequenz des genutzten Frequenzbandes.

Durch UWB wird die Position der Gelenke und somit die Stellung des Skeletts detektiert. Aus der Gelenkstellung können auch entsprechende Bewegungen detektiert werden. UWB wird ggf. durch ein Kamerasystem (stereo camera) unterstützt.

Eine erfindungsgemäße Kombination der vorgenannten Verfahren ist EIT und UWB.

In einer besonderen Ausführung der Erfindung ist der Aktuator geeignet, auf den Körper des Benutzers mittels Vibrationen einzuwirken.

In diesem Fall liegt der Aktuator am Körper des Benutzers an, um die Vibration direkt zu übertragen. Die Vibration ist vor allem sinnvoll, um dem Benutzer z.B. eine Fehlstellung des Körpers bzw. Körperbereichen zu signalisieren. Die Vibration kann in unterschiedlichen Stärkegraden erfolgen, um den Grad der Fehlstellung anzuzeigen.

Bevorzugt umfasst der Aktuator einen Detektor zur Erfassung der Position und/ oder der Bewegung des Aktuators relativ zum Körper des Benutzers und/oder relativ zur Umgebung.

Neben der Detektion von Muskel- und Gewebekontraktionen, kann die Position des Aktuators selbst in Bezug auf den Körper und/ oder zur Umgebung bestimmt werden. Sofern mehrere Aktuatoren verwendet werden, können die Positionen zueinander in Bezug gesetzt werden, um relative Positionen und Bewegungen zu erfassen.

Neben den oben beschriebenen UWB kann auch eine inertiale Messeinheit IMU (inertial measurement unit) verwendet werden.

IMU ist eine räumliche Kombination mehrerer Inertialsensoren wie z.B.

Beschleunigungssensoren und Drehratensensoren. Sie stellt die sensorische Messeinheit
eines Trägheitsnavigationssystems dar und kann die Bewegungsdynamik erfassen.

Vorliegend werden die Bewegungen des Körpers bzw. Köperteilen gemessen.

In vorteilhafter Weise ist der Aktuator geeignet, über den Zustand eines Bereichs des Körpers des Benutzers Messdaten zu erheben, an dem der Aktuator angeordnet ist.

Die Messung kann durch die genannten EIT, EMT, UWB und IMU Verfahren erfolgen

In einer besonders vorteilhaften Ausführung ist der Aktuator in einen vom Benutzer am Körper tragbaren Träger, insbesondere ein Kleidungsstück, eingearbeitet und/oder an diesem befestigt.

Der Aktuator kann hierbei auch als sogenannter Wearable in Form eines Bandes, einer Manschette bzw. eines Gurts geformt sein, der über dem betreffenden Körperbereich angeordnet wird.

In einer vorteilhaften Ausführung umfasst das System einen Vorhersagebereich, der zur Erstellung eines Bewegungsdatensatzes aufgrund einer Vielzahl von Messdaten auf Basis des Datenverarbeitungsmodells geeignet ist.

Der Vorhersagebereich ist in der Regel eine lokale Recheninstanz mit entsprechenden Rechenkapazitäten, um die essentiellen Verarbeitungsschritte für die Analyse und Vorhersage der Bewegung und der Erstellung eines entsprechenden Bewegungsdatensatz durchzuführen. Im Vorhersagebereich werden die im Detektionsbereich detektierten Messdaten empfangen, dekomprimiert, verarbeitet.

Das Machine Learning kann gegebenenfalls durch Analysen/ Implementierungen in Convolutional Neural Networks (CNN) erfolgen. In diesem Fall werden im Vorhersagebereich mit Hilfe des durch convolutional neural networks (CNN) erstellten Datenverarbeitungsmodells die Analysen bzw. Bewegungsdatensätze berechnet.

Durch die geographische Nähe und die dedizierte Ressourcenbereitstellung lassen sich so die benötigten niedrigen Latenzen zwischen Sensorik und Aktorik realisieren.

Daneben kann der Vorhersagebereich geeignet sein, das Datenverarbeitungsmodell zu speichern.

Der Vorhersagebereich weist hierzu entsprechende Speichermedien auf, z.B. lokale Speicher wie Festplatten oder nicht-lokale Speicher, wie den Zugriff auf Cloud-Speicher.

Bevorzugt ist der Vorhersagebereich geeignet, Messdaten vom Detektionsbereich zu empfangen und zu verarbeiten.

Die Messdaten können darüber hinaus an den Modellierungsbereich zur Aktualisierung des Datenverarbeitungsmodells weitergegeben werden.

In vorteilhafter Weise ist der Vorhersagebereich geeignet, aus vom Detektionsbereich empfangenen Messdaten einen Bewegungsablauf des Benutzers zu erkennen.

Insbesondere aus einer Vielzahl von Messdaten, die eine räumliche Veränderung des Körpers messen, kann im Vorhersagebereich auf einen entsprechenden Bewegungsablauf geschlossen werden. Neben einen oder mehrerer Bewegungsabläufe können auch Gesten oder Bewegungen des Benutzers erkannt werden. Z.B. kann eine Kontraktion einer bestimmten Muskelgruppe auf ein Anheben eines Gegenstands hinweisen, oder eine bestimmte Winkelstellung des Beins des Benutzers lässt auf eine Aufwärts- oder Abwärtsbewegung, z.B. ein Springen, schließen. Die Gestenerkennung kann die Datenverarbeitung im Datenverarbeitungsmodell unterstützen oder vereinfachen, und die Vorhersagegenauigkeit oder -zuverlässigkeit von Bewegungen verbessern.

Die Bearbeitung der Messdaten umfasst eine Dekomprimierung der Messdaten im Vorhersagebereich.

Hierbei werden die durch den Detektionsbereich komprimierten Messdaten wieder dekomprimiert, z.B. im Falle von Bildern (Aufnahme von Muskelkontraktionen) werden die Bildschichten wieder zusammengefügt.

Bevorzugt ist der Vorhersagebereich geeignet, aus dem Bewegungsdatensatz ein ganzes oder teilweises Körpermodell des Benutzers und/oder aktuelle und/oder zukünftige Bewegungsabläufe des Benutzers zu visualisieren.

Die Visualisierung kann intern innerhalb des Systems oder extern erfolgen. So kann z.B. die Visualisierung der Muskelaktivitäten auf einem Endgerät (beispielsweise einem Tablet) angezeigt werden.

In einer besonderen Ausführung umfasst das System wenigstens einen Aktuator, der geeignet ist, auf Basis des Bewegungsdatensatzes auf den Körper des Benutzers und/oder auf eine vom Benutzer verwendete Vorrichtung einzuwirken.

Der Aktuator dient der Einwirkung auf den Benutzer oder dessen Trainingsgerät, um eine bestimmte Reaktion des Benutzers auszulösen bzw. anzustoßen. Dies kann rein visuell erfolgen, so dass dem Benutzer angezeigt wird, wie z.B. der Körper bzw. Körperbereiche, bewegt werden müssen, um eine gewünschte Bewegung zu erzielen bzw. zu optimieren. Alternativ dazu kann z.B. im Wege der direkten Einflussnahme, z.B. durch Warntöne, Summen, elektrische oder mechanische Impulse, etc., eine Reaktion des Benutzers angestoßen werden, so dass dieser z.B. eine körperliche Fehlstellung korrigiert.

Daneben kann der Aktuator auch den aktuellen Zustand (Ist-Zustand) des Körpers messen und ein entsprechendes Feedback an den Vorhersagebereich geben. Auf diese Weise wird der Körperzustand dauerhaft überwachen, z.B. wenn eine bestimmte Köperhaltung eingehalten werden soll.

Eine Einflussnahme eines Aktuators auf das vom Benutzer benutzte Sportgerät kann beispielsweise darin bestehen, dass das Sportgerät eine gewünschte oder vorhergesagte Bewegung des Benutzers unterstützt oder dieser entgegenwirkt, je nach Trainingsziel.

Die Erfindung hat weiter ein Verfahren zur Erkennung und Vorhersage von Bewegungen eines Benutzers zum Gegenstand, umfassend die folgenden Verfahrensschritte:
a) Bereitstellen eines erfindungsgemäßen Systems.
b) Erfassung von Messdaten durch Messungen mittels wenigstens eines Sensors am Körper des Benutzers und/ oder mittels des Aktuators, ggf. auch durch Messungen an einem benutzten Sportgerät.
c) Weiterleiten der Messdaten an den Modellierungsbereich.
d) Erstellung eines Datenverarbeitungsmodells aufgrund der Messdaten mittels Machine Learning.
e) Einwirkung auf Körper und/oder Rückmeldung mittels des Aktuators.

Das Machine Learning kann durch Analysen in Convolutional Neural Networks (CNN) erfolgen.

Wie bereits oben beschrieben ist CNN ein Konzept zur maschinellen Verarbeitung von Bild- oder Audiodaten.

Ein weiteres Verfahren zur Erkennung und Vorhersage von Bewegungen eines Benutzers, umfasst die folgenden Verfahrensschritte:
a) Bereitstellen eines erfindungsgemäßen Systems.
b) Erfassung von Messdaten durch Messungen mittels des Sensors am Körper des Benutzers und/ oder mittels des Aktuators, ggf. auch durch Messungen an einem benutzten Sportgerät.
c) Weiterleiten der Messdaten an den Vorhersagebereich.
d) Erstellung eines Bewegungsdatensatzes unter Verwendung des Datenverarbeitungsmodells.
e) Bearbeitung und/oder Speicherung und/oder Darstellung des Bewegungsdatensatzes im Vorhersagebereich.
f) Einwirkung auf den Körper und/oder Rückmeldung mittels des Aktuators.

Bevorzugt umfasst das Verfahren den weiteren Verfahrensschritt:
g) Anpassung des Datenverarbeitungsmodells unter Verwendung der an den Modellierungsbereich weitergeleiteten Messdaten.

Durch die Anpassung des Datenverarbeitungsmodells kann eine fortwährende Aktualisierung und Verbesserung des Datenverarbeitungsmodells stattfinden.

Die Erfassung der Messdaten im Detektionsbereich zur Erfassung des Körperzustands des Benutzers erfolgt mittels EIT (elektrische Impedanz Tomographie) und/ oder EMT und/ oder UWB.

Mittels EIT (wie oben bereits eingehend beschrieben) werden mittels Messung von Impedanz- bzw. Kapazitätsänderungen zwischen Messpunkten mehrere Einzelbilder aus unterschiedlichen Schichten des zu betrachtenden Teils des Körpers erstellt und daraus ein detailliertes Gesamtbild erstellt.

Durch EMG wird die elektrische Muskelaktivität anhand von Aktionsströmen der Muskeln gemessen und ggf. (graphisch) dargestellt.

UWB ist eine Form der Nahbereichsfunkkommunikation bei Nutzung großer Frequenzbereiche.

Bevorzugt umfasst das Verfahren den weiteren Verfahrensschritt:
Einwirkung des Aktuators des bereitgestellten Systems auf Basis des Bewegungsdatensatzes auf den Körper des Benutzers und/oder auf eine vom Benutzer verwendete Vorrichtung, z.B. ein Sportgerät.

Der Aktuator dient der Umsetzung des Bewegungsmodells durch entsprechende Einwirkung auf den Körper des Benutzers. Dies kann rein visuell erfolgen, so dass dem Benutzer angezeigt wird, wie z.B. der Körper bzw. Körperbereiche, bewegt werden müssen, um eine gewünschte Bewegung zu erzielen. Oder im Wege der direkten Einflussnahme, z.B. durch Warntöne, Summen etc. um z.B. den Benutzer auf eine körperliche Fehlstellung hinzuweisen.

### KURZE BESCHREIBUNG DER FIGUREN

- Figur 1: Schematische Darstellung der einzelnen Komponenten des erfindungsgemäßen Systems;
- Figur 2: Darstellung der Anordnung von Sensoren am Ober- und Unterarm eines Benutzers;
- Figur 3: Schematische Darstellung der punktuellen Anordnung von einem oder mehreren Aktuatoren am Körper eines Benutzers;
- Figur 4A: Schematische Darstellung der punktuellen Anordnung am Körper eines Benutzers gemäß Figur 3 in Bezug auf ein Koordinatensystem zur Bestimmung der Position von einem oder mehreren Aktuatoren
- Figur 4B: Schematische Darstellung der Bestimmung der Gelenkbewegungen aufgrund der punktuellen Anordnung am Körper eines Benutzers gemäß Figur 4A.

### BESCHREIBUNG EINES BEVORZUGTER AUSFÜHRUNGSBEISPIELE

Anhand nachfolgender Ausführungsbeispiele soll die Erfindung näher erläutert werden.

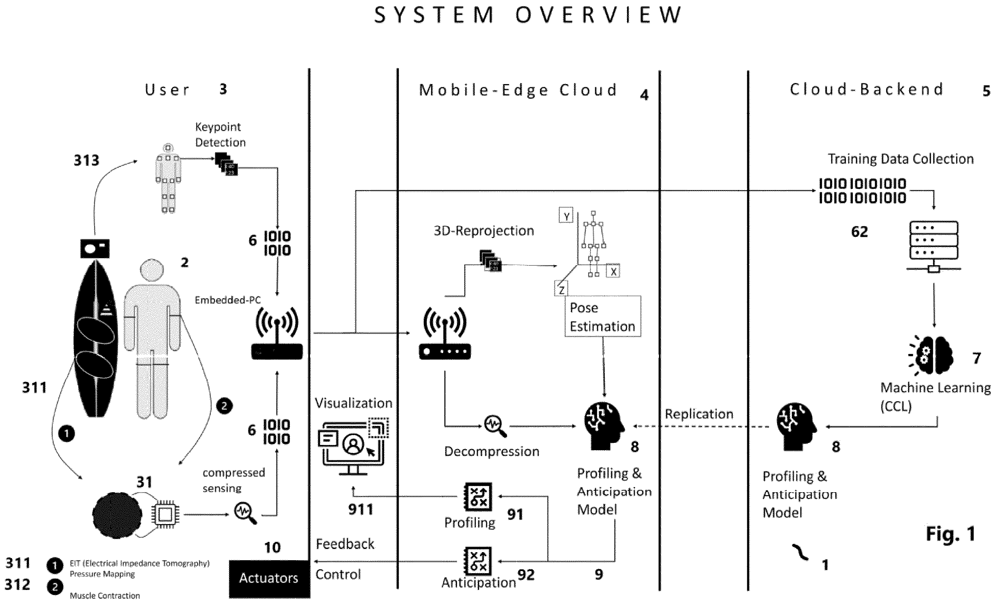

Aus Figur 1 wird ersichtlich, dass das erfindungsgemäße System 1 grundsätzlich in drei Teile, nämlich User 3, Mobile-Edge Cloud 4 und Cloud-Backend 5 aufgeteilt ist, wobei User 3 dem Detektionsbereich, Mobile-Edge Cloud 4 dem Vorhersagebereich und Cloud-Backend 5 dem Modellierungsbereich entspricht.

Bei dem Detektionsbereich 3 als User- bzw. Benutzerebene, ist der Sensor 31/ die Sensorik möglichst kompakt am Benutzer 2 selbst und/oder an einem Interaktionsgegenstand angebracht und/oder in letzterem integriert. Der Sensor 31 erfasst mittels EIT (elektrischer Impedanz Tomographie /Electronical Impedance Tomography) Messdaten 6 über den Körper des Benutzers 2, z.B. durch Druckdarstellung 311 (pressure mapping) oder Muskelkontraktionen 312.

Bei der Datenerhebung werden mehrere Einzelbilder aus unterschiedlichen "Blickwinkeln" geschichtet und es entsteht auf diese Weise ein detailliertes dreidimensionales (3D) Gesamtbild. Dieses Verfahren wird beispielsweise zur Ermittlung der Gewichtsverteilung eines Surfers auf seinem Surfbrett verwendet, wobei über eine Manschette, die vorzugsweise an den Beinen und/oder Armen des Benutzers angebracht ist, ein Muskelquerschnittsbild (beispielsweise der Muskeln des Oberschenkels) erzeugt wird. So können die Muskelkontraktionen gemessen und auf Bewegungen rückgeschlossen werden. Die Anzahl der Bilder, die erstellt wird, ist recht erheblich, entsprechend groß sind auch die Datenmengen. Deshalb werden die Sensorwerte über ein "compressed sensing" Verfahren abgegriffen, was die Datenakquise stark beschleunigt, weil die Daten bereits komprimiert gemessen werden.

Neben der Druckdarstellung 311 oder der Messung der Muskelkontraktion 312 ist daneben oder kumulativ die Messung von bestimmten Schlüsselpunkten (keypoints) 313 des Körpers des Benutzers 2 möglich. Hierbei ist vor allem die (Gelenk-) Stellung/Anordnung der Extremitäten (z.B. Arme, Beine, Rumpf und Kopf) von Bedeutung, um Rückschlüsse auf die Körperstellung des Benutzers 2 zu erhalten.

Durch die verschiedenen Messungen 311, 312, 313 werden Messdaten 6 erfasst.

Im Detektionsbereich 3 findet somit nur die Datenakquise und minimale Vorverarbeitung (Filtern, Komprimieren zur Größenreduktion, Fusionieren) statt. Die Messdaten 6 werden an die Mobile-Edge Cloud 4 und/ oder den Cloud Backend-Server 5 übertragen.

Im Cloud Backend-Server bzw. Modellierungsbereich 5 werden die übermittelten Messdaten 6 gesammelt und zu Trainingsdatensets 62 für die Erstellung der Machine-Learning Modelle zusammengestellt. Im Rahmen dieses Trainings werden die CNNs (convolutional neural networks) für die Mobile-Edge-Cloud Instanzen erstellt und nach Fertigstellung dort aktualisiert.

Mittels Machine Learning 7 wird ein Datenverarbeitungsmodel 8 erstellt, welches im Anschluss an den Vorhersagebereich/ die Mobile-Edge Cloud 4 weitergeleitet wird.

Der mittlere Bereich, der Verarbeitungsbereich/ Mobile-Edge Cloud 4, ist eine lokale Recheninstanz mit entsprechenden Rechenkapazitäten, um die essentiellen Verarbeitungsschritte für die Analyse und Vorhersage der Bewegungen des Körpers des Benutzers 2 durchführen. Im Verarbeitungsbereich 4 werden die vom Detektionsbereich 3 bereitgestellten Messdaten 6 empfangen, dekomprimiert, verarbeitet und die Analysen (Profiling & Anticipation) mit Hilfe des durch convolutional neural networks (CNN) erstellten Profiling & Anticipation Modells errechnet. Bei der Dekompression werden die Bildschichten zusammengefügt und zur Visualisierug der Körperaktivitäten auf einem Endgerät 911 (beispielsweise Tablet) angezeigt.

Auf diese Weise wird das vom Modellierungsbereich 5 bereitgestellte Datenverarbeitungsmodell 8 weiter zu einem Bewegungsdatensatz 9 (profiling/anticipation) erarbeitetet. Dieser kann einerseits visualisiert 91 werden und andererseits zur Einflussnahme 92 über einen Aktuator 10 auf den Körper des Benutzers 2 oder des Sportgeräts dienen. Es ist auch die Visualisierung von vorhergesagten Bewegungen. Der Aktuator 10 kann auf den Körper des Benutzers 2 einwirken, diesen aber auch kontrollieren und entsprechende Rückmeldung geben. Von daher ist der Aktuator 10 auch im Detektionsbereich 3 angesiedelt.

Durch die räumliche Nähe und die dedizierte Ressourcenbereitstellung lassen sich niedrige Latenzen zwischen Sensorik und Aktorik realisieren.

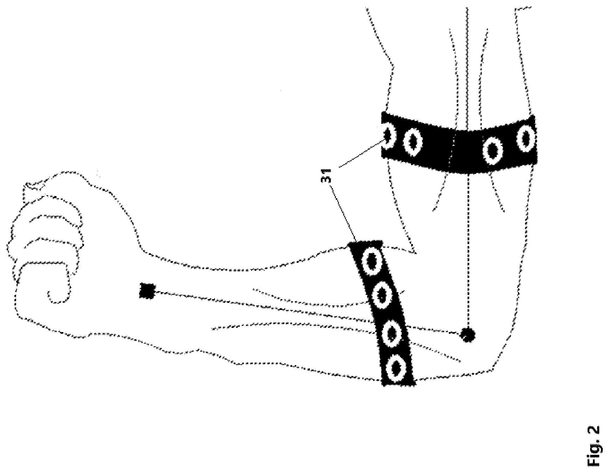

Fig. 2 zeigt die Anordnung von zwei Sensoren 31, die jeweils auf dem Unterarm und dem Oberarm eines Benutzers angeordnet sind,

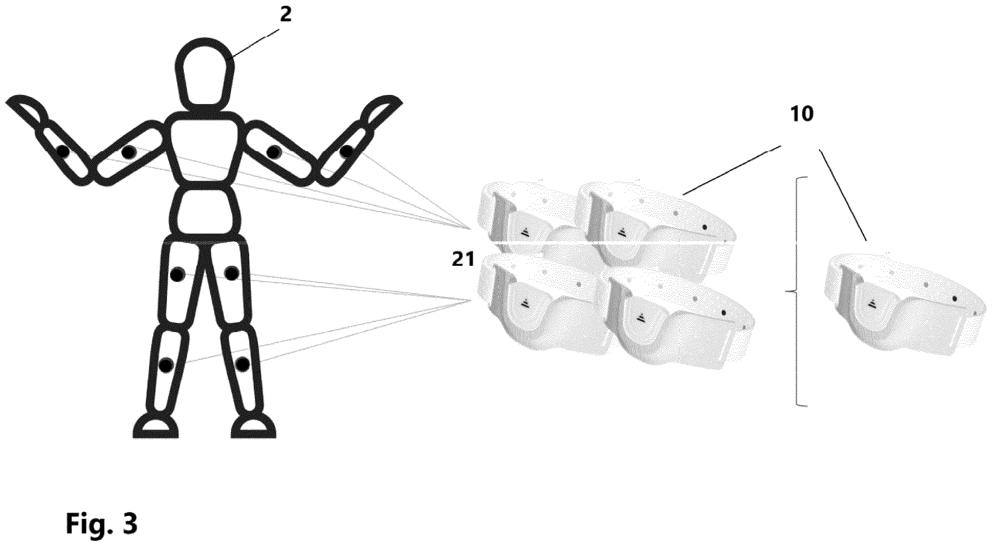

Fig. 3 zeigt die Anordnung eines oder mehrerer Sensoren/Aktuatoren 10 (in Form von sog. Wearables) an Punkten des Körpers (z.B. an Ober-, Unterarm und Ober-, und Unterschenkel). Die Wearables sind in Form von Bändern, die an den jeweiligen Punkten 21 am Körper eines Benutzers 2 angeordnet werden, ausgebildet. Durch EIT und/ oder EMG wird die Muskel- und Gewebeaktivität gemessen. Über UWB kann die Stellung der Gelenke detektiert werden. Sofern z.B. je ein Aktuator 10 an Ober- und Unterarm angebracht ist, kann die Stellung des Gelenks zwischen Ober- und Unterarm gemessen werden und somit auch die Stellung und Bewegung des Arms gemessen werden. Über die Aktuatoren 10 kann dem Benutzer auch ein Feedback, z.B. über Vibrationen, gegeben werden, z.B. wenn eine Fehlstellung oder eine Fehlbewegung vorliegt.

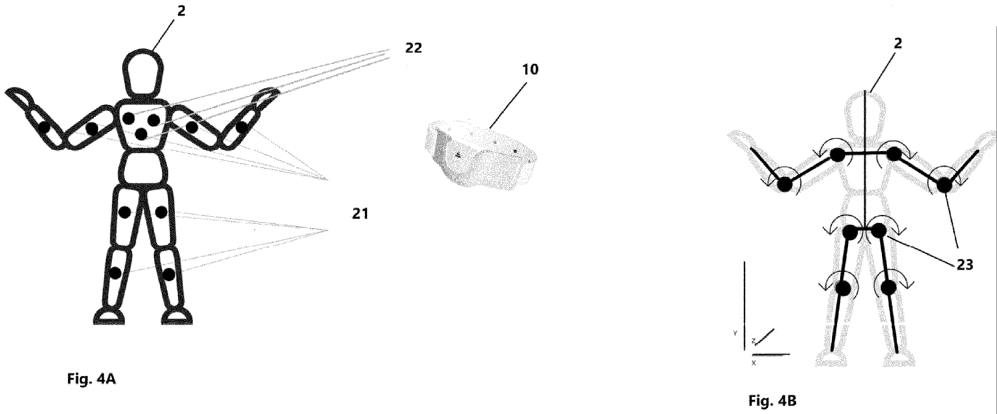

Fig. 4A zeigt die Detektion der Gelenkstellung mittels Aktuatoren (Wearable) 10 die an Körperpunkten 21 entsprechend der Fig. 3 angebracht sind. Ein Wearable weist in der Regel einen integrierten IMU und einen UWB Chip auf. Ein Dreipunkt-Ankersystem (in Bezug auf den Bezug auf den Körper oder der Umgebung) trianguliert die absolute Position des Wearables im dreidimensionalen Raum in anpassbaren Abständen.

Über die drei Ankerpunkte 22 wird mittels Luftschnittstelle des UWB ein Referenzsystem aufgebaut und über TDoA (Time Difference of Arrival) eine Multilaterationsberechnung zur Bestimmung der X-, Y- und Z-Koordinaten des Wearable durchgeführt. Dadurch wird in zyklischen Abständen eine absolute Position der einzelnen Wearables (z.B. ca. alle 5 Sekunden) durchgeführt. Dazwischen wird über die auf den Aktuatoren 10 angeordneten IMUs die relative Bewegung der Gliedmaßen im Raum gemessen. So werden konstant zuverlässige Motion Capturing Daten über jede mit Wearables ausgestattete Gliedmaße erhalten. Die Ankerpunkte lassen sich dabei bedarfsgerecht setzen. Also beispielsweise direkt am Körper, oder am Spielfeldrand etc.

Fig 4B zeigt die relative Bewegung der Gelenke, dargestellt durch die Punkte 23, die durch IMU gemessen werden. Der IMU Sensor Drift wird durch das UWB Koordinatensystem erhalten.

## Patentansprüche

1. System (1) zur Erkennung und Vorhersage von Bewegungen eines Benutzers (2), umfassend einen Detektionsbereich (3) mit mindestens einem Sensor (31), einen Modellierungsbereich (5), einen Vorhersagebereich (4) und einen Aktuator (10), wobei der Sensor (31) geeignet ist, über den Körperzustand des Benutzers (2) Messdaten (6) zu erheben und an den Modellierungsbereich (5) weiterzuleiten, wobei die Erfassung des Körperzustands und/oder einer Körperregion des Benutzers (2) mittels EIT (Elektrische Impedanz Tomographie) und UWB (Ultra-wideband) Echtzeit-Lokalisierung erfolgt, wobei zur Erfassung des Körperzustands und/oder der Körperregion durch Messung der internen elektrischen Impedanz ein Schnittbild zur Gewebezusammensetzung innerhalb der untersuchten Körperregion innerhalb der Impedanzverteilung erzeugt wird, und mehrere Einzelbildern aus unterschiedlichen Schichten des zu betrachtenden Objekts und Zusammenführung zu einem detaillierten dreidimensionalen Gesamtbild erstellt werden, und wobei der Modellierungsbereich (5) geeignet ist, aufgrund der Messdaten (6) durch Machine Learning ein Datenverarbeitungsmodel (7) zur Berechnung eines aktuellen und/oder zukünftigen Körperzustands des Benutzers (2) zu erstellen, und wobei der Aktuator (10) geeignet ist, auf Basis des Datenverarbeitungsmodels (7) auf den Körper des Benutzers (2) und/oder auf eine vom Benutzer (2) verwendete Vorrichtung einzuwirken, wobei der Vorhersagebereich (4) zur Erstellung eines Bewegungsdatensatzes (8) aufgrund einer Vielzahl von Messdaten (6; 61) auf Basis des Datenverarbeitungsmodells (7) geeignet ist, und geeignet ist, aus dem Bewegungsdatensatz (8) ein ganzes Körpermodell des Benutzers zu visualisieren.

2. System (1) zur Erkennung und Vorhersage von Bewegungen eines Benutzers (2) nach Anspruch 1, wobei EIT der Erfassung der Muskelaktivität und/oder des Zustands des Körpergewebes, und die Detektion mittels EMG der Erfassung der Muskelaktivität und/oder des Zustands des Körpergewebes und UWB Echtzeit-Lokalisierung der Erfassung von Gelenkstellungen und/oder der räumlichen Anordnung der Knochen des Benutzers dient.

3. System (1) zur Erkennung und Vorhersage von Bewegungen eines Benutzers (2) nach einem der vorhergehenden Ansprüche 1 bis 2, wobei der Aktuator (10) geeignet ist, auf den Körper des Benutzers mittels Vibrationen einzuwirken.

4. System (1) zur Erkennung und Vorhersage von Bewegungen eines Benutzers (2) nach einem der vorhergehenden Ansprüche 1 bis 3, wobei der Aktuator (10) einen Detektor zur Erfassung der Position und/ oder der Bewegung des Aktuators (10) relativ zum Körper des Benutzers und/oder relativ zur Umgebung umfasst.

5. System (1) zur Erkennung und Vorhersage von Bewegungen eines Benutzers (2) nach einem der vorhergehenden Ansprüche 1 bis 4, wobei der Aktuator (10) geeignet ist, über den Zustand eines Bereichs des Körpers des Benutzers (2) Messdaten (61) zu erheben, an dem der Aktuator (10) angeordnet ist.

6. System nach einem der vorhergehenden Ansprüche 1 bis 5, wobei der Aktuator (10) in einen vom Benutzer am Körper tragbaren Träger, insbesondere ein Kleidungsstück, eingearbeitet und/oder an diesem befestigt ist

7. System (1) zur Erkennung und Vorhersage von Bewegungen eines Benutzers (2) nach Anspruch 1, wobei der Vorhersagebereich (4) geeignet ist, Messdaten (6; 61) vom Detektionsbereich (3) und/ oder vom Aktuator (10) zu empfangen und zu verarbeiten.

8. System (1) zur Erkennung und Vorhersage von Bewegungen eines Benutzers (2) nach Anspruch 7, wobei der Vorhersagebereich (4) geeignet ist, aus von vom Detektionsbereich (3) und/ oder von dem Aktuator (10) empfangenen Messdaten (6; 61) einen Bewegungsablauf des Benutzers (2) zu erkennen.

9. System (1) zur Erkennung und Vorhersage von Bewegungen eines Benutzers (2) nach einem der vorhergehenden Ansprüche 7 bis 8, wobei der Vorhersagebereich (4) geeignet ist, aus dem Bewegungsdatensatz (8) ein ganzes oder teilweises Körpermodell des Benutzers (2) und/oder Bewegungsabläufe des Benutzers (2) zu visualisieren.

10. System (1) zur Erkennung und Vorhersage von Bewegungen eines Benutzers (2) nach einem der vorhergehenden Ansprüche 1 bis 10, wobei der Aktuator (10) geeignet ist, auf Basis des Bewegungsdatensatzes (8) auf den Körper des Benutzers (2) und/oder auf eine vom Benutzer (2) verwendete Vorrichtung einzuwirken.

11. Verfahren zur Erkennung und Vorhersage von Bewegungen eines Benutzers (2), umfassend die folgenden Verfahrensschritte:
a) Bereitstellen eines Systems (1) nach einem der Ansprüche 1 bis 10.
b) Erfassung von Messdaten (6; 61) durch Messungen mittels wenigstens eines Sensors (3) am Körper des Benutzers (2) und/ oder mittels des Aktuators (10).
c) Erstellung von mehreren Einzelbildern mittels Messung von Impedanzänderungen aus unterschiedlichen Schichten des zu betrachtenden Teils des Körpers und Schichtung zu einem detaillierten dreidimensionalen Gesamtbild.
c) Weiterleiten der Messdaten (6; 61) an den Modellierungsbereich (5) oder den Vorhersagebereich (4).
d) Erstellung eines Datenverarbeitungsmodells (7) aufgrund der Messdaten (6; 61) mittels Machine Learning.
e) Erstellung eines Bewegungsdatensatzes (8) unter Verwendung des Datenverarbeitungsmodells (7).
f) Bearbeitung und/oder Speicherung und/oder Darstellung des Bewegungsdatensatzes (8) im Vorhersagebereich (4),
g) Einwirkung auf Körper und/oder Rückmeldung mittels des Aktuators (10).

12. Verfahren zur Erkennung und Vorhersage von Bewegungen eines Benutzers (2) nach Anspruch 11, umfassend den weiteren Verfahrensschritt:
a) Anpassung des Datenverarbeitungsmodells (7) unter Verwendung der an den Modellierungsbereich (5) weitergeleiteten Messdaten (6; 61).

13. Verfahren zur Erkennung und Vorhersage von Bewegungen eines Benutzers (2) nach einem der vorhergehenden Ansprüche 11 bis 12, umfassend den weiteren Verfahrensschritt:
b) Einwirkung des Aktuators (10) des bereitgestellten Systems (1) auf Basis des Bewegungsdatensatzes (8) auf den Körper des Benutzers (2) und/oder auf eine vom Benutzer (2) verwendete Vorrichtung.

## Claims

1. System (1) for identifying and predicting movements of a user (2), comprising a detection unit (3) with at least one sensor (31), a modeling unit (5), a prediction unit (4), and an actuator (10), wherein the sensor (31) is capable of collecting measurement data (6) through the user's (2) physical condition and to transmit it to the modeling unit (5), wherein the detection of the user's (2) physical condition and/or a body region is performed using EIT (Electrical Impedance Tomography) and UWB (Ultra-wideband) real-time localization, wherein, for the detection of the physical condition and/or the body region by measuring the internal electrical impedance, a cross-sectional image of the tissue composition within the examined body region is generated within the impedance distribution, and multiple individual images from different layers of the object under examination are created and merged into a detailed three-dimensional overall image, and wherein the modeling unit (5) is capable of using machine learning to create a data processing model (7) based on the measurement data (6) for calculating a current and/or future physical state of the user (2), and wherein the actuator (10) is configured for acting on the user's (2) body and/or on a device used by the user (2), wherein the prediction unit (4) is configured to generate a motion data set (8) based on a plurality of measurement data (6; 61) using the data processing model (7), and is configured to visualize a full-body model of the user from the motion data set (8).

2. System (1) for identifying and predicting movements of a user (2) according to claim 1, wherein EIT is used to measure muscle activity and/or the state of body tissue, and the detection by means of EMG serves to record muscle activity and/or the state of the body tissue, and UWB real-time localization serves to record joint positions and/or the spatial arrangement of the user's bones.

3. System (1) for identifying and predicting movements of a user (2) according to claims 1 to 2, wherein the actuator (10) is configured for act on the user's body by means of vibrations.

4. System (1) for identifying and predicting movements of a user (2) according to any one of claims 1 to 3, wherein the actuator (10) comprises a detector for identifying the position and/or the movement of the actuator (10) relative to the user's body and/or relative to the environment.

5. System (1) for identifying and predicting movements of a user (2) according to any one of claims 1 to 4, wherein the actuator (10) is adapted to collect measurement data (61) regarding the condition of a region of the user's (2) body on which the actuator (10) is located.

6. System according to any one of claims 1 to 5, wherein the actuator (10) is incorporated into and/or attached to a carrier that can be worn on the user's body, in particular a garment.

7. System (1) for identifying and predicting movements of a user (2) according to claim 1, wherein the prediction unit (4) is configured for receive and process measurement data (6; 61) from the detection unit (3) and/or from the actuator (10).

8. System (1) for identifying and predicting movements of a user (2) according to claim 7, wherein the prediction unit (4) is configured for detect a sequence of movements of the user (2) based on measurement data (6; 61) received from the detection unit (3) and/or from the actuator (10).

9. System (1) for identifying and predicting movements of a user (2) according to any one of claims 7 to 8, wherein the prediction unit (4) is configured for visualize, from the motion data set (8), a complete or partial body model of the user (2) and/or motion sequences of the user (2).

10. System (1) for identifying and predicting movements of a user (2) according to any one of claims 1 to 10, wherein the actuator (10) is configured for act on the body of the user (2) and/or on a device used by the user (2) based on the motion data set (8).

11. Method for identifying and predicting movements of a user (2), comprising the following method steps:
a) Providing a system (1) according to any one of claims 1 to10.
b) Acquiring measurement data (6; 61) through measurements using at least one sensor (3) on the user's (2) body and/or using the actuator (10).
c) Generating of multiple individual images by measuring changes in impedance from different layers of the part of the body being examined and layering these into a detailed three-dimensional composite image.
c) Forwarding the measurement data (6; 61) to the modeling unit (5) or the prediction unit (4).
d) Generating of a data processing model (7) based on the measurement data (6; 61) using machine learning.
e) Generating of a motion data set (8) using the data processing model (7).
f) Processing and/or storing and/or displaying the motion data set (8) in the prediction unit (4),
g) Acting on the body and/or providing feedback via the actuator (10).

12. Method for identifying and predicting movements of a user (2) according to claim 11, comprising the further method step:
a) Adapting the data processing model (7) using the measurement data (6; 61) forwarded to the modeling unit (5).

13. Method for identifying and predicting movements of a user (2) according to any one of claims 11 to 12, comprising the further method step:
b) Acting the actuator (10) of the provided system (1) on the body of the user (2) and/or on a device used by the user (2) based on the motion data set (8).

## Revendications

1. Système (1) destiné de détection et de prévision des mouvements d'un utilisateur (2), comprenant une zone de détection (3) équipée d'au moins un capteur (31), une zone de modélisation (5), une zone de prévision (4) et un actionneur (10), le capteur (31) étant apte à collecter des données de mesure (6) sur l'état corporel de l'utilisateur (2) et de les transmettre à la zone de modélisation (5), la détection de l'état corporel et/ou d'une région corporelle de l'utilisateur (2) s'effectuant au moyen de l'EIT (tomographie par impédance électrique) et de l'UWB (ultra-large bande) et de la localisation en temps réel, la détection de l'état corporel et/ou de la région du corps par mesure de l'impédance électrique interne générant une image en coupe de la composition tissulaire au sein de la région corporelle examinée dans la distribution d'impédance, et plusieurs images individuelles provenant de différentes couches de l'objet à examiner étant créées et fusionnées en une image globale tridimensionnelle détaillée, et dans lequel la zone de modélisation (5) est adaptée pour créer, à partir des données de mesure (6) par apprentissage automatique, un modèle de traitement de données (7) permettant de calculer un état corporel actuel et/ou futur de l'utilisateur (2), et dans lequel l'actionneur (10) est adapté pour agir sur le corps de l'utilisateur (2) et/ou sur un dispositif utilisé par l'utilisateur (2), la zone de prévision (4) étant adaptée pour créer un ensemble de données de mouvement (8) à partir d'une pluralité de données de mesure (6 ; 61) sur la base du modèle de traitement de données (7), et étant adaptée pour visualiser, à partir de l'ensemble de données de mouvement (8), un modèle corporel complet de l'utilisateur.

2. Système (1) destiné de détection et de prévision des mouvements d'un utilisateur (2) selon la revendication 1, dans lequel l'EIT (électro-imagerie) sert à la détection de l'activité musculaire et/ou à l's sur l'état des tissus corporels, et la détection par EMG sert à la mesure de l'activité musculaire et/ou de l'état des tissus corporels, tandis que l'UWB et la localisation en temps réel servent à la mesure des positions articulaires et/ou de la disposition spatiale des os de l'utilisateur.

3. Système (1) destiné de détection et de prévision des mouvements d'un utilisateur (2) selon l'une des revendications 1 à 2 précédentes, l'actionneur (10) étant adapté pour agir sur le corps de l'utilisateur au moyen de vibrations,

4. Système (1) destiné de détection et de prévision des mouvements d'un utilisateur (2) selon l'une des revendications 1 à 3 précédentes, dans lequel l'actionneur (10) comprend un détecteur pour détecter la position et/ou le mouvement de l'actionneur (10) par rapport au corps de l'utilisateur et/ou par rapport à l'environnement.

5. Système (1) destiné de détection et de prévision des mouvements d'un utilisateur (2) selon l'une des revendications 1 à 4 précédentes, dans lequel l'actionneur (10) est adapté pour collecter des données de mesure (61) sur l'état d'une zone du corps de l'utilisateur (2) sur laquelle l'actionneur (10) est disposé.

6. Système selon l'une des revendications 1 à 5 précédentes, dans lequel l'actionneur (10) est intégré et/ou fixé à un support pouvant être porté sur le corps par l'utilisateur, en particulier un vêtement

7. Système (1) destiné de détection et de prévision des mouvements d'un utilisateur (2) selon la revendication 1, dans lequel la zone de prévision (4) est adaptée pour recevoir et traiter des données de mesure (6 ; 61) provenant de la zone de détection (3) et/ou de l'actionneur (10).

8. Système (1) destiné de détection et de prévision des mouvements d'un utilisateur (2) selon la revendication 7, dans lequel la zone de prévision (4) est apte à détecter une séquence de mouvements de l'utilisateur (2) à partir de données de mesure (6 ; 61) reçues de la zone de détection (3) et/ou de l'actionneur (10) .

9. Système (1) destiné de détection et de prévision des mouvements d'un utilisateur (2) selon l'une des revendications 7 à 8 précédentes, dans lequel la zone de prévision (4) est adaptée pour visualiser, à partir de l'ensemble de données de mouvement (8), un modèle corporel complet ou partiel de l'utilisateur (2) et/ou des séquences de mouvements de l'utilisateur (2).

10. Système (1) destiné de détection et de prévision des mouvements d'un utilisateur (2) selon l'une des revendications précédentes 1 à 10, dans lequel l'actionneur (10) est adapté pour agir, sur la base de l'ensemble de données de mouvement (8), sur le corps de l'utilisateur (2) et/ou sur un dispositif utilisé par l'utilisateur (2).

11. Procédé de détection et de prévision des mouvements d'un utilisateur (2), comprenant les étapes suivantes :
a) mise à disposition d'un système (1) selon l'une des revendications 1 à 10 ;
b) Acquisition de données de mesure (6 ; 61) par des mesures effectuées au moyen d'au moins un capteur (31) placé sur le corps de l'utilisateur (2) et/ou au moyen de l'actionneur (10).
c) Génération de plusieurs images individuelles à partir de la mesure des variations d'impédance dans différentes couches de la partie du corps à examiner, puis assemblage de ces couches pour obtenir une image globale tridimensionnelle détaillée.
c) Transmission des données de mesure (6 ; 61)vers la zone de modélisation (5) ou la zone de prévision (4).
d) Création d'un modèle de traitement des données (7) à partir des données de mesure (6 ; 61) à l'aide de l'apprentissage automatique.
e) Création d'un ensemble de données de mouvement (8) à l'aide du modèle de traitement des données (7).
f) Traitement et/ou stockage et/ou représentation de l'ensemble de données de mouvement (8) dans la zone de prévision (4),
g) Action sur le corps et/ou retour d'information au moyen de l'actionneur (10).

12. Procédé de détection et de prévision des mouvements d'un utilisateur (2) selon la revendication 11, comprenant l'étape supplémentaire suivante :
a) Adaptation du modèle de traitement de données (7) à l'aide des données de mesure (6 ; 61) transmises à la zone de modélisation (5).

13. Procédé de détection et de prévision des mouvements d'un utilisateur (2) selon l'une des revendications précédentes 11 à 12, comprenant l'étape supplémentaire suivante :
b) action de l'actionneur (10) du système (1) mis à disposition sur le corps de l'utilisateur (2) et/ou sur un dispositif utilisé par l'utilisateur (2), sur la base de l'ensemble de données de mouvement (8).
